## Europäisches Patentamt
## European Patent Office
## Office européen des brevets

(19)

(11) Veröffentlichungsnummer: **0 194 453**
**B1**

(12) **EUROPÄISCHE PATENTSCHRIFT**

(45) Veröffentlichungstag der Patentschrift:
04.01.89

(51) Int. Cl.⁴: **A 41 B 13/02**

(21) Anmeldenummer: **86101706.5**

(22) Anmeldetag: **11.02.86**

(54) Windel.

(30) Priorität: **14.05.85 DE 8514294 U**
**11.02.85 DE 8503728 U**

(43) Veröffentlichungstag der Anmeldung:
**17.09.86 Patentblatt 86/38**

(45) Bekanntmachung des Hinweises auf die Patenterteilung:
**04.01.89 Patentblatt 89/1**

(84) Benannte Vertragsstaaten:
**AT BE CH DE FR GB IT LI NL SE**

(56) Entgegenhaltungen:
**GB-A-918 064**
**GB-A-1 067 730**
**US-A-4 397 646**
**US-A-4 402 690**
**US-A-4 516 975**

(73) Patentinhaber: **Exquisit Kurt Götz, Aumühle**
**Container Terminal, D-8700 Würzburg (DE)**

(72) Erfinder: **Götz, Kurt, Aumühle Container Terminal,**
**D-8700 Würzburg (DE)**

(74) Vertreter: **Schuster, Thomas, Dipl.- Phys.,**
**Maximilianstrasse 58, D-8000 München 2 (DE)**

### Beschreibung

Die Erfindung betrifft eine Windel mit einer für Nässe undurchlässigen Außenhaut, mit einem waschbaren, Nässe durchlässigen Innenstoff, wobei Außenhaut und Innenstoff umlaufend miteinander vernäht und zu den an Bauch und Rücken des Windelträgers zur Anlage kommenden Enden hin unter Bildung von Laschen gegenüber dem Beinanlagebereich seitlich verbreitert sind, wobei die Außenhaut über diese Enden übersteht und mit einer zwischen Außenhaut und Innenstoff eingeschlossenen Saugeinlage.

Derartige Windeln sind bekannt. Sie sind im Gegensatz zu Einmal- und oder Wegwerfwindeln mit einer waschbaren Innenlage versehen, so daß sie mehrmals verwendet werden können. Die Innenlage wird bei solchen Windeln mit der für Nässe undurchlässigen Außenhaut umlaufend vernäht. Bei gattungsgemäßen Windeln steht die Nässe undurchlässige Außenhaut über die Innenlage mit einem Abschnitt nach außen über. Dieser überstehende Abschnitt der Außenhaut, die im allgemeinen aus einer dünnen Kunststoffhaut besteht, legt sich bei der Verwendung der Windel am Bauch und am Rücken des Windelträgers an und verhindert, daß die Nässe oder die Feuchtigkeit der Windel mit den Kleidern des Windelträgers in Kontakt kommt. Der Abschnitt wirkt somit als Nässestop.

Nachteilig bei den bekannten derartigen Nässestops ist es aber, daß sich die Abschnitte, die den Nässestop bilden sollen, beim Waschen verknittern bzw. sich in der Art einer Krepprolle zusammenziehen, so daß nach einmaligem Einsatz der Windel in den meisten Fällen dieser Abschnitt seine Funktion als Nässestop nicht mehr zuverlässig erfüllen kann.

Ein weiterer Nachteil dieser bekannten Windeln liegt darin, daß die Abdichtung im Beinausschnittsbereich, d.h. im mittleren Bereich der Windel, an dem diese gegenüber den Beinen abdichten soll, nur unzureichend möglich ist. Bei bekannten Windeln bildet die in dem Beinbereich Außenhaut, Innenstoff und Saugeinlage zusammenfassende Naht im wesentlichen die äußere Begrenzung der Windel. Da die Windel bei ihrer Verwendung gerade in diesem Bereich am stärksten umgebogen ist, entstehen offene Räume zwischen Windel und Bein, so daß die Dichtwirkung der Windel gerade in diesem kritischen Bereich nur unzureichend ist.

Aufgabe der Erfindung ist es daher eine Windel zu schaffen, die mit einem ringsum wirkenden zuverlässigen Nässestop ausgestattet ist, der auch nach mehrmaligem Waschen der Windel in seiner Funktion noch voll wirksam sein kann.

Diese Aufgabe wird mit einer Windel der eingangs genannten Art gelöst, die dadurch gekennzeichnet ist, daß die Außenhaut an den an Bauch und Rücken des Trägers zur Anlage kommenden Enden mit einem Umbug über den Innenstoff übersteht und daß die Außenhaut und der Innenstoff im Beinanlagebereich zu beiden Seiten hin gegenüber der Saugeinlage nach außen seitlich verbreitert verlaufen und daß ein elastisches Zugelement in dem verbreiterten Mittelbereich vorgesehen ist, welches die Außenhaut und den Innenstoff in diesem Bereich zusammenzieht.

Durch die mittels des Umbugs gebildete zweilagige Anordnung der Außenhaut wird diese versteift und rollt sich daher beim Waschen nicht zusammen. Die Funktion des Umbugs als Nässestop bleibt daher auch nach mehrmaligem Waschen erhalten, so daß eine zuverlässige Dichtwirkung am Bauch-und Rückenanlagebereich sichergestellt werden kann.

Um die Dichtwirkung im Beinbereich zu erhöhen, sieht die Erfindung eine in diesem Bereich seitliche Verbreiterung von Außenhaut und Innenstoff vor. Das in diesem verbreiterten Bereich zu beiden Seiten jeweils angeordnete elastische Zugelement zieht im Beinbereich Außenhaut und Innenstoff durch eine in Längserstreckung der Windel wirkende Kraft zusammen, so daß Außenhaut und Innenstoff gegenüber dem Bereich, in dem die Saugeinlage vorliegt, eine jeweils seitlich nach oben stehende Wandung bilden, die sich automatisch an die Beine des Windelträgers anlegt. Das Zugelement, das auch über die gesamte Seitenlänge der Windel verlaufen kann, kommt dabei etwas unter Spannung und sorgt somit für einen optimalen Sitz bzw. eine bestmögliche Abdichtung auch im Beinbereich.

In vorteilhafter Weiterbildung der Erfindung ist vorgesehen, daß das elastische Zugelement ein auf den Innenstoff aufgenähtes, gummielastisches Stoffband ist. Gegenüber einer möglichen Lösung, bei der das Gummielement zwischen Außenhaut und Innenstoff als Gummiband eingenäht ist, bietet diese vorteilhafte Ausgestaltung den Vorteil, daß das gummielastische Stoffband trotz der hervorgerufenen Zugwirkung nicht zu einer Kräuselung bzw. Wellung an der Innenseite der Windel führt, was die Trageeigenschaften der Windel verbessert, weil die Scheuerwirkung der Windel an den Beinen dadurch stark vermindert werden kann.

Das Einnähen des Gummibandes kann so erfolgen, daß eine Doppelnaht entlang beider Längsränder gleichzeitig genäht wird, so daß sich beim Einnähen das Gummiband nicht verzieht.

Eine weitere Ausgestaltung der Erfindung sieht vor, daß die Außenhaut zur Bildung des Umbugs zur Innenlage hin umgefaltet ist und daß der umgefaltete Teil zwischen der Innenlage und der darüberliegenden Außenhaut endet. Dies bringt außer der optimalen Nässestopwirkung noch den weiteren Vorteil mit sich, daß die Trageeigenschaften verbessert werden, weil die Kante, mit der der umgefaltete Teil endet, dann nicht mit dem Körper des Trägers in Berührung kommt.

In weiterer vorteilhafter Ausgestaltung ist vorgesehen, daß die Außenhaut, der umgefaltete Teil und die Innenlage durch eine einzige Naht miteinander verbunden sind. Dies bringt hinsichtlich der Herstellung den Vorteil mit sich, daß sich die Innenlage nur mit einer einzigen Naht mit der am Nahtstellenbereich dann zweischichtigen Außenhaut verbinden läßt.

Eine weitere Ausgestaltung der Erfindung sieht vor, daß die Saugeinlage aus zwei Schichten besteht, wobei sich wenigstens eine Schicht bis in den Bereich der Laschen erstreckt. Die Windel nützt damit auch den im Bereich der Laschen vorhandenen Raum zwischen Außenhaut und Innenstoff aus und verwendet diesen Raum ebenfalls zum Aufsaugen der durch den Innenstoff eindringenden Nässe. Die Windel besitzt dann eine große Saugfähigkeit.

Bei einer konkreten, bevorzugten Ausgestaltung erstreckt sich eine erste Schicht, die auf der Außenhautseite liegt, bis in den Bereich der Laschen, und es ist vorgesehen, daß sich eine zweite auf der Innenstoffseite liegende Schicht mit etwa parallel verlaufenden Seiten zwischen den Enden estreckt.

Durch diese Ausgestaltung ist die Windel im Mittelteil, in dem beim bestimmungsgemäßen Einsatz der Windel am meisten Nässe entsteht, verstärkt saugfähig, d.h. sie weist eine größere Dicke auf als im Bereich der Laschen. Die Nässe verteilt sich dann gleichmäßig über die gesamte saugfähige Fläche.

Die beiden Schichten können jeweils aus saugfähigem Material gleicher oder unterschiedlicher Stärke bestehen. Die Stärke der Filzmaterialien kann je nach Saugfähigkeit des Materials und Größe der Windel frei gewählt werden. Die Verwendung gleich dicker Filzmaterialien sowohl für die erste als auch für die zweite Lage erleichtert allerdings die Herstellung.

Außer Filzmaterialien können auch andere Web-, Vlies- oder Strickmaterialien als Saugeinlage Verwendung finden.

Eine weitere vorteilhafte Ausgestaltung der Erfindung sieht vor, daß die erste Lage am Rand umlaufend mit den Rändern des Innenstoffs und der Außenhaut vernäht ist. Dadurch wird die erste Lage bei der Herstellung der Windel gleichzeitig mit dem Zusammenähen des Innenstoffs und der Außenhaut fixiert und unverschieblich festgelegt.

Bevorzugt wird auch die zweite Schicht wenigstens im Bereich der Enden durch Nähte gegenüber der ersten Schicht und dem Innenstoff fixiert. Dann kann es insbesondere beim Waschen der Windel nicht zu einer Verschiebung und dadurch zu einer Beeinträchtigung der Paßform der Windel kommen.

In einer weiteren sehr vorteilhaften Ausgestaltung ist der Windel eine zusätzliche Saugeinlage zugeordnet, die in die Windel einlegbar ist. Durch diese Ausgestaltung wird es möglich, die in der Windel fest eingenähten Saugschichten relativ dünn zu halten, so daß die Windel nach dem Waschen schneller trocknet. Durch die zusätzlich einlegbare Saugeinlage kann aber trotzdem sichergestellt werden, daß beispielsweise bei Nacht die dann besonders benötigte hohe Saugfähigkeit der Windel sichergestellt ist. Die zusätzliche Saugeinlage kann aus Zellstoff oder aus Mischstoff mit hoher Saugfähigkeit bestehen. Sie wird angepaßt an die Form der Windel geschnitten.

Die Erfindung wird im folgenden anhand eines in den Zeichnungen dargestellten Ausführungsbeispiels weiter erläutert und beschrieben.

Figur 1    zeigt eine aufgeklappte, erfindungsgemäße Windel in einer Ansicht auf die Innenseite,

Figur 2    zeigt einen Schnitt durch die Windel entlang der Linie II-II der Figur 1,

Figur 3    zeigt einen Schnitt durch die in Figur 1 dargestellte Windel entlang der Linie III-III,

Figur 4    zeigt eine aufgeklappte Windel mit einer zusätzlichen Saugeinlage.

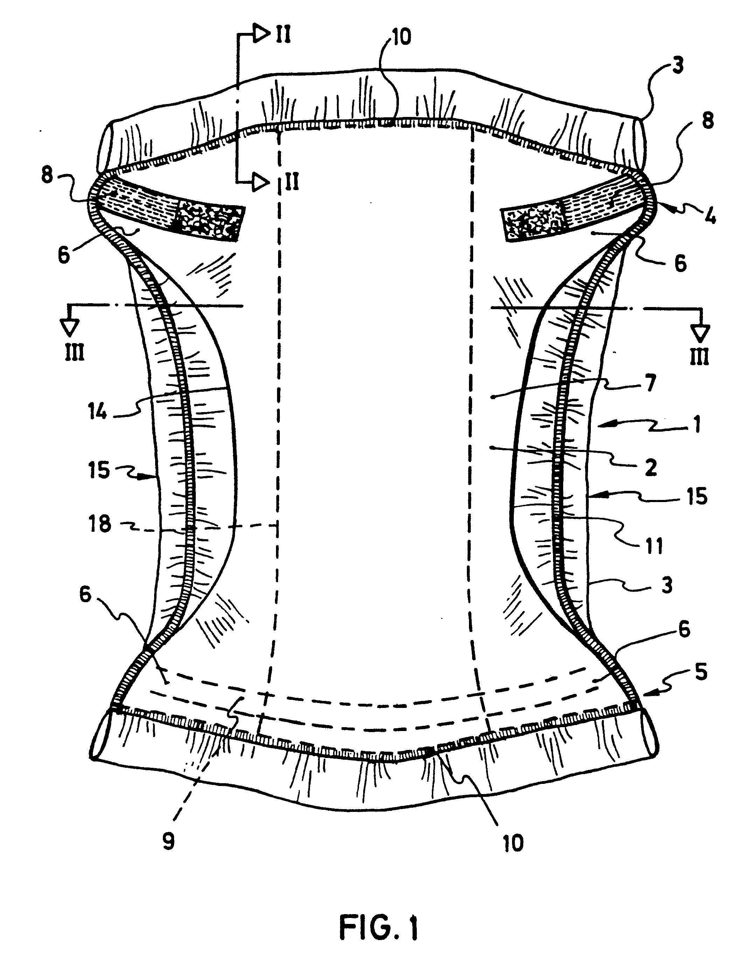

Die in Figur 1 dargestellte Windel ist im Ganzen mit 1 bezeichnet. An den Enden 4 bzw. 5, mit denen die Windel zur Anlage in Brust- bzw. Rückenbereich des Windelträgers kommt, weist die Windel gegenüber ihrem Mittelbereich, mit dem sie in der Beingegend anliegt, seitlich verbreiterte Laschen 6 auf, an denen sie mit Klettverschlüssen 8 und einem entsprechenden Gegenstück 9, dessen Verlauf auf der Außenseite der Windel gestrichelt angedeutet ist, verschließbar ist.

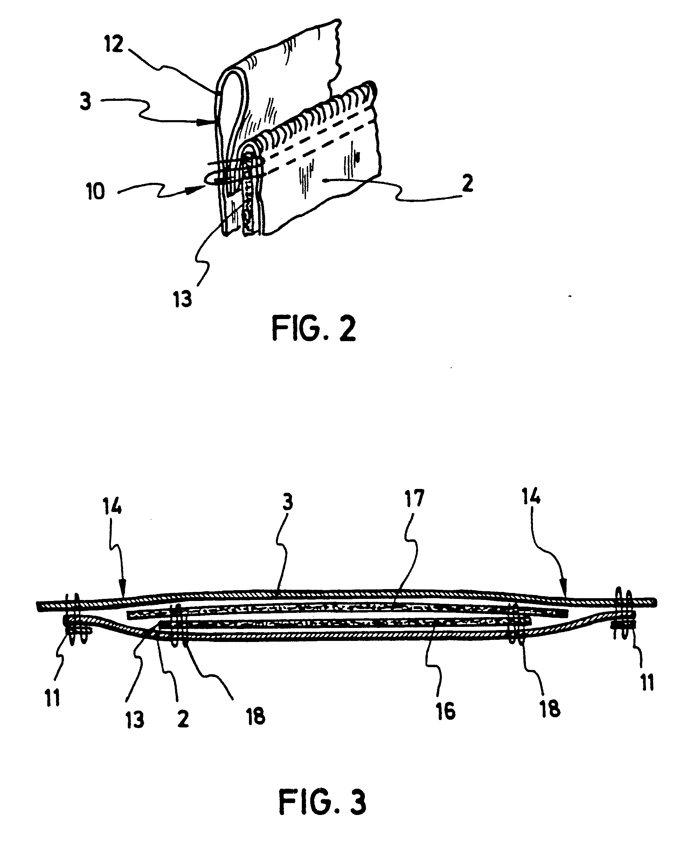

Wie aus Figur 1 im Zusammenhang mit Figur 2 und 3 zu sehen ist, weist die Windel eine Außenhaut 3 auf, die beispielsweise aus einem dünnen Kunststoffmaterial wie Nylon oder dgl. hergestellt sein kann. Die Innenseite der Windel wird von einem Innenstoff 2, beispielsweise aus einem Baumwollmaterial, gebildet.

Innenstoff und Außenhaut haben im wesentlichen dieselbe Form und sind durch eine umlaufende Naht 10 miteinander verbunden. Die nur an den Enden dargestellte Naht 10 wird an den Seiten der Windel entlang des noch näher zu beschreibenden elastischen Zugelementes 11 fortgesetzt.

Die Außenhaut 3 steht im Bereich der sich gegenüberliegenden Enden 4 und 5 über das durch die Naht 10 definierte Ende des Innenstoffes 2 nach außen über.

Die Außenhaut 3 wirkt im Bereich dieses überstehenden Abschnittes als Nässestop und legt sich am Bauch bzw. am Rücken des Trägers unter Bildung eines feuchtigkeitsdichten Abschlusses an.

Wie insbesondere Figur 2 deutlich erkennen läßt, bildet die Außenhaut 3 in diesem überstehenden Bereich einen Umbug 12. Dieser Umbug verhindert ein Zusammenrollen beim Waschvorgang, so daß die Nässestopwirkung dieses überstehenden Umbugs 12 auch nach mehrmaligem Waschen erhalten bleibt.

Wie ebenfalls Fig. 2 deutlich zeigt, ist im dargestellten Ausführungsbeispiel die Außenhaut 3 nach innen zur Innenlage 2 hin umgefaltet. Das vordere Ende dieses umgefalteten Teils wird zwischen der eigentlichen Außenhaut 3 und dem Innenstoff bzw. Saugeinlage 13 gelegt, so daß die Außenhaut 3, der Innenstoff 2 und die Saugeinlage 13 unter Bildung des Umbugs 12 durch eine einzige Naht 10 zusammengezogen bzw. miteinander vernäht werden können.

In Figur 1 deutet desweiteren die zwischen den Laschen 6 jeweils seitlich angedeutete, durchgezogene Linie 14 die Begrenzung der Saugeinlage 13 an. Im Zusammenhang mit dem in Figur 3 dargestellten Schnitt läßt sich somit erkennen, daß über diesen mittleren oder auch Beinanlagebereich 7 die Außenhaut 3 und der Innenstoff 2 seitlich gegenüber der Saugeinlage 13 verbreitert ist. Der Innenstoff 2 ist bis in den Bereich eines zwischen den Laschen 6 auf jeder Seite verlaufenden gummielastischen Zugelementes 11 geführt und dort zusammen mit dem Zugelement und der Außenhaut 3 vernäht.

Die Außenhaut steht noch etwas weiter seitlich über das elastische Zugelement 11 nach außen ab.

Das elastische Zugelement 11 ist bei diesem Ausführungsbeispiel ein gummielastisches, eine gewisse Breite aufweisendes Stoffband, welches auf der Innenseite der Windel zusammen mit dem Innenstoff und der Außenhaut 3 vernäht wird. Ein solches gummielastisches Stoffband sorgt auch in seiner zusammengezogenen Stellung für eine glatte Innenfläche, so daß die Trageigenschaften der Windel nicht durch Wellungen oder dgl. beeinträchtigt werden.

Wenn die Windel aus der in Figur 1 dargestellten gestreckten oder aufgeklappten Position zur Anlage an den Windelträger gebracht wird, zieht sich das Gummiband zusammen und sorgt dafür, daß der verbreiterte, über die Begrenzung 14 überstehende Bereich von Innenstoff und Außenhaut sich eng um die Beingegend des Windelträgers legt. Dieser verbreiterte Bereich 15 sorgt somit für eine zuverlässige Abdichtung auch in der Beingegend des Windelträgers.

Das gummielastische Stoffband 11 kann, wie das in Figur 1 dargestellt ist, über die gesamte Länge zwischen den Endbereichen 4 und 5 der Windel verlaufen. Es ist aber auch möglich, das Gummiband 11 nur in dem Bereich verlaufen zu lassen, in dem die Begrenzung 14 der Saugeinlage zwischen den Laschen 6 verjüngt ist.

Wie insbesondere Figur 3 erkennen läßt, ist bei diesem Ausführungsbeispiel eine zweischichtige Saugeinlage 13 vorgesehen.

Die erste Schicht 17, die an die Innenseite der Außenhaut 3 angrenzt, nimmt den in Figur 1 durch die Begrenzung 14 angedeuteten Verlauf, d.h., sie erstreckt sich auch über die Laschen 6 bis zu der Naht 10 an den beiden Enden 4 bzw. 5 der Windel.

Auf diese erste Schicht 19 ist eine zweite Schicht 16 gelegt, die mit der Innenseite des Innenstoffes 2 zur Anlage kommt.

Diese zweite Schicht 16 nimmt im wesentlichen den durch die Naht 18 in Figur 1 dargestellten Verlauf, d.h., sie ist im wesentlichen als rechteckförmiger Streifen ausgebildet, der zwischen den Enden 4 und 5 der Windel verläuft.

Durch diese Ausgestaltung besitzt die Windel über den in Längsrichtung verlaufenden Mittelteil eine hohe Flüssigkeitsaufnahmefähigkeit und verteilt die aufgesaugte Flüssigkeit gleichmäßig über die gesamte Saugeinlage 13.

Bei dem gezeigten Ausführungsbeispiel sind die beiden Schichten 16 und 17 durch die Naht 18 mit der Innenhaut 2 in Längsrichtung vernäht. Dadurch ist die Lage der beiden Schichten zuverlässig fixiert und es kann nicht zu Verschiebungen während des Waschvorganges kommen.

Es ist jedoch im Rahmen der Erfindung genauso möglich, beispielsweise nur die erste Schicht 17 entlang ihrer Begrenzung 14 mit dem Innenstoff 2 zu vernähen und die zweite Schicht 16 dann lediglich im Bereich der Enden durch Quernähte zu fixieren.

In dem gezeigten Ausführungsbeispiel ist weiterhin die Schichtdicke der beiden Schichten 16 und 17 etwa gleich groß dargestellt. Es ist aber genauso möglich, für die Schichten 16 und 17 Materialien, beispielsweise Filzstoffe, mit unterschiedlichen Dicken zu verwenden.

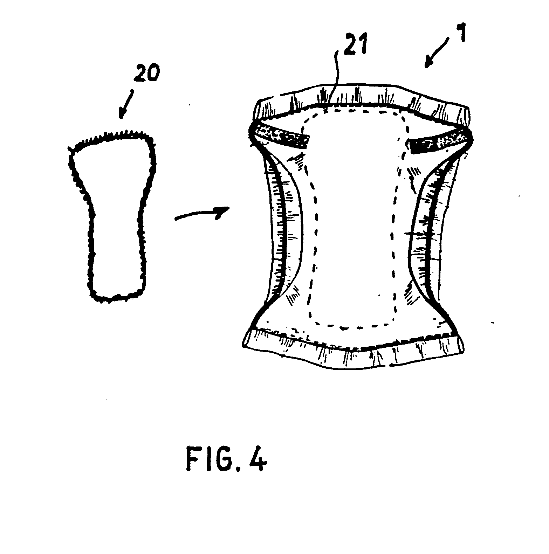

In Figur 4 ist der Windel 1 eine zusätzliche Saugeinlage 20 zugeordnet, die aus Zellstoff oder einem Mischstoff mit hoher Saugfähigkeit besteht und in die Windel so eingelegt werden kann, daß ihre Begrenzung etwa den Verlauf der gestrichelten Kontur 21 nimmt. Diese zusätzliche Saugeinlage erhöht die Saugfähigkeit der Windel und kann beispielsweise über Nacht zusätzlich in die Windel eingelegt werden, so daß sich die von der Windel mit Saugeinlage aufgenommene Feuchtigkeitsmenge auf die fest eingenähte Saugeinlage in der Windel und auf die eingelegte Einlage 20 verteilt. Nach dem Waschen trocknet die Windel und auch die Saugeinlage schneller, als wenn die festeingenähte Saugeinlage in der Windel entsprechend dick gewählt werden würde.

**Patentansprüche**

1. Windel (1) mit einer für Nässe undurchlässigen Außenhaut (3), mit einem waschbaren, für Nässe durchlässigen Innenstoff (2), wobei Außenhaut und Innenstoff umlaufend miteinander vernäht und zu den an Bauch und Rücken des Windelträgers zur Anlage kommenden Enden (4, 5) hin unter Bildung von Laschen (6) gegenüber einem Beinanlagebereich (7) seitlich verbreitert sind und wobei die Außenhaut über diese Enden übersteht und mit einer zwischen Außenhaut und Innenstoff eingeschlossenen Saugeinlage (13), dadurch gekennzeichnet, daß die Außenhaut (3) an den an Bauch und Rücken des Trägers zur Anlage kommenden Enden (4, 5) mit einem Umbug (12) über den Innenstoff (2) übersteht und daß die Außenhaut und der Innenstoff im Beinanlagebereich (7) zu beiden Seiten hin gegenüber der Saugeinlage (13) nach außen seitlich verbreitert verlaufen und daß ein elastisches Zugelement (11) in dem verbreiterten Mittelbereich (15) vorgesehen ist, welches die Außenhaut (3) und den Innenstoff (2) in diesem Bereich zusammenzieht.

2. Windel nach Anspruch 1, dadurch gekennzeichnet, daß das elastische Zugelement ein auf den Innenstoff aufgenähtes, gummielastisches Stoffband (11) ist.

3. Windel nach Anspruch 1 oder 2, dadurch gekennzeichnet, daß die Außenhaut (3) zur Bildung des Umbugs (12) zum Innenstoff (2) hin umgefaltet ist und daß der umgefaltete Teil zwischen dem Innenstoff (2) und der darüberliegenden Außenhaut (3) endet.

4. Windel nach Anspruch 3, dadurch gekennzeichnet, daß die Außenhaut (3), der umgefaltete Teil und der Innenstoff (2) durch eine einzige Naht miteinander verbunden sind.

5. Windel nach wenigstens einem der vorangegangenen Ansprüche, dadurch gekennzeichnet, daß sich die Saugeinlage (13) auch in den Bereich der Laschen (6) erstreckt.

6. Windel nach Anspruch 5, dadurch gekennzeichnet, daß die Saugeinlage (13) aus zwei Schichten (16, 17) besteht, wobei sich wenigstens eine Schicht (17) bis in den Bereich der Laschen (6) erstreckt.

7. Windel nach Anspruch 6, dadurch gekennzeichnet, daß sich eine erste Schicht (17), die an der Außenhautseite liegt, bis in den Bereich der Laschen (6) erstreckt und daß sich eine zweite Schicht (16), die auf der Innenstoffseite liegt mit etwa parallel verlaufenden Seiten zwischen den Enden (4, 5) erstreckt.

8. Windel nach Anspruch 6 oder 7, dadurch gekennzeichnet, daß die beiden Schichten (16, 17) jeweils aus saugfähigem Filzmaterial gleicher oder unterschiedlicher Stärke bestehen.

9. Windel nach wenigstens einem der Ansprüche 6 bis 8, dadurch gekennzeichnet, daß die erste Schicht (17) umlaufend mit dem Innenstoff (2) und der Außenhaut (3) vernäht ist.

10. Windel nach wenigstens einem der Ansprüche 6 bis 9, dadurch gekennzeichnet, daß die zweite Schicht (16) wenigstens im Bereich der Enden (4, 5) durch Nähte gegenüber der ersten Schicht (17) und dem Innenstoff (2) fixiert ist.

11. Windel nach wenigstens einem der vorangegangenen Ansprüche, gekennzeichnet durch eine zusätzliche Saugeinlage (20), die in die Windel (1) einlegbar ist.

**Claims**

1. A diaper (1) comprising: a moisture-impervious outer skin (3); a washable moisture-pervious inner material (2), the outer skin and inner material being sewn together around their periphery and being widened laterally to form flaps (6) opposite a leg-contacting zone (7) towards the ends (4, 5) which engage the belly and back of the diaper wearer, the outer skin projecting beyond such ends; and an absorbent insert (13) included between the outer skin and the inner material, characterised in that the outer skin (3) projects in a loop (12) beyond the inner material (2) at the ends (4, 5) which engage the wearer's belly and back, the outer skin and inner material widen laterally outwards towards both sides from the absorbent insert (13) in the leg-contacting zone (7), and a resilient draw or pull element (11) is provided in the widened central zone (15) and draws the outer skin (3) and the inner material (2) together in the latter zone.

2. A diaper according to claim 1, characterised in that the resilient draw element (11) is an elastomeric strip of material sewn to the inner material.

3. A diaper according to claim 1 or 2, characterised in that the outer skin (3) is folded over towards the inner material (2) to form the loop (12) and the folded-over part terminates between the inner material (2) and the outer skin (3) thereabove.

4. A diaper according to claim 3, characterised in that the outer skin (3), the folded-over part and the inner material (2) are interconnected by a single seam.

5. A diaper according to at least one of the previous claims, characterised in that the absorbent insert (13) also extends to near the flaps (6).

6. A diaper according to claim 5, characterised in that the absorbent insert (13) comprises two layers (16, 17), at least one layer (17) extending to near the flaps (6).

7. A diaper according to claim 6, characterised in that a first layer (17) which is disposed on the outer skin side extends to near the flaps (6) and a second layer (16) disposed on the inner material side extends by way of substantially parallel sides between the ends (4, 5).

8. A diaper according to claim 6 or 7, characterized in that the two layers (16, 17) are made of absorbent felt material which may be of the same or of different thickness.

9. A diaper according to at least one of claims 6 - 9, characterised in that the first layer (17) is sewn peripherally to the inner material (2) and outer skin (3).

10. A diaper according to at least one of claims 6 - 9, characterized in that the second layer (16) is secured relatively to the first layer (17) and inner material (2) by seams at least near the ends (4, 5).

11. A diaper according to at least one of the previous claims, characterised by an additional absorbent insert (20) introducible into the diaper (1).

## Revendications

1. Lange (1) comportant une peau extérieure (3) imperméable à l'humidité, ainsi qu'un matériau intérieur (2) lavable, perméable à l'humidité, étant précisé que la peau extérieure et le matériau intérieur sont cousus ensemble sur la périphérie et vont en s'élargissant latéralement, par rapport à une zone (7) d'appui sur les jambes, en direction des extrémités (4, 5) qui viennent en appui sur le ventre et le dos du porteur du lange, en formant des pattes (6); et étant précisé que la peau extérieure déborde au-delà de ces extrémités, et comportant aussi une couche absorbante (13) enfermée entre la peau extérieure et le matériau intérieur, caractérisé en ce que, aux extremités (4, 5) qui viennent en appui contre le ventre et le dos du porteur, la peau extérieure (3) déborde, par un rabat (12), au-delà du matériau intérieur (2); et en ce que, dans la zone (7) d'appui sur les jambes, la peau extérieure et le matériau intérieur vont en s'élargissant latéralement vers l'extérieur par rapport à la couche absorbante (13), en direction des deux côtés; et en ce qu'il est prévu, dans la zone médiane élargie (15) un élément élastique de tirage (11) qui resserre dans cette zone la peau extérieure (3) et le matériau intérieur (2).

2. Lange selon la revendication 1, caractérisé en ce que l'élément élastique de tirage est un ruban (11) à élasticité de type caoutchouc, cousu sur le matériau intérieur.

3. Lange selon la revendication 1 ou 2, caractérisé en ce que la peau extérieure (3) est repliée en direction du matériau intérieur (2) pour former le rabat (12); et en ce que la partie repliée se termine entre le matériau intérieur (2) et la peau extérieure (3) située par-dessus.

4. Lange selon la revendication 3, caractérisé en ce que la peau extérieure (3), la partie repliée et le matériau intérieur (2) sont réunis ensemble par une unique couture.

5. Lange selon au moins l'une des revendications précédentes, caractérisé en ce que la couche absorbante (13) s'étend également dans la zone des pattes (6).

6. Lange selon la revendication 5, caractérisé en ce que la couche absorbante (13) est constituée de deux couches (16, 17), étant précisé qu'une couche (17) au moins s'étend jusque dans la zone des pattes (6).

7. Lange selon la revendication 6, caractérisé en ce qu'une première couche (17), située du côté de la peau extérieure, s'étend jusque dans la zone des pattes (6); et en ce qu'une seconde couche (16), située du côté du matériau intérieur, s'étend entre les deux extrémités (4, 5) avec des côtés dirigés à peu près parallèlement.

8. Lange selon la revendication 6 ou 7, caractérisé en ce que les deux couches (16, 17) sont chacune constituées d'un matériau absorbant du type feutre de même épaisseur ou d'épaisseur différente.

9. Lange selon au moins l'une des revendications 6 à 8, caractérisé en ce que la première couche (17) est cousue sur la périphérie avec le matériau intérieur (2) et la peau extérieure (3).

10. Lange selon au moins l'une des revendications 6 à 9, caractérisé en ce que la seconde couche (16) est fixée au moins dans la zone des extrémités (4, 5) par des coutures vis-à-vis de la première couche (17) et du matériau intérieur (2).

11. Lange selon au moins l'une des revendications précédentes, caractérisé par une couche absorbante supplémentaire (20) que l'on peut insérer dans le lange (1).

FIG. 1

**FIG. 2**

**FIG. 3**

FIG. 4